# EUROPEAN PATENT APPLICATION

(11) **EP 4 603 034 A1**
(43) Date of publication of application: **20.08.2025**
(21) Application number: 23876847.7
(22) Date of filing: 28.09.2023
(51) Int. Cl.: A61B 17/16

(54) **COSTOTOME**

(30) Priority: 13.10.2022 ES 202230880
(71) Applicant: Universidad de Las Palmas de Gran Canaria, 35001 Las Palmas de Gran Canaria (ES)
(72) Inventor: MARTEL FUENTES, Óscar, 35012 Las Palmas De Gran Canaria Las Palmas (ES); YÁNEZ SANTANA, Alejandro, 35010 Las Palmas De Gran Canaria Las Palmas (ES); PÉREZ ALONSO, David, 35003 Las Palmas De Gran Canaria Las Palmas (ES); DE LEÓN RODRÍGUEZ, Ignacio, 38720 San Andrés y Sauces Sta. Cruz Tenerife (ES)
(74) Representative: Ungria López, Javier
(86) International application number: PCT/ES2023/070578
(87) International publication number: WO 2024/079369

(57) **Abstract**

The present invention relates to a device for breaking a patient's ribs, which is designed to break a plurality of consecutive ribs in a single manouevre and allows safe and controlled cutting.

## Description

### OBJECT OF THE INVENTION

The present invention relates to a device for breaking a patient's ribs, configured to break a plurality of consecutive ribs in a single manoeuvre. This single manoeuvre allows for safe and controlled cutting.

The present device is useful in surgical procedures on a patient, in particular in surgical procedures involving the thorax, and more particularly in surgical procedures aimed at correcting *pectus excavatum* in a patient.

Said device, in an operational situation, performs a single percutaneous manoeuvre through a single incision in addition to those made in the surgical procedure in question, this additional incision being aimed at inserting the device into the patient's thorax.

The present device therefore allows the patient's ribs to be broken in a simultaneous manoeuvre to obtain not only a more efficient procedure, but also a shorter and less painful post-operative period for the patient.

### BACKGROUND OF THE INVENTION

In the technical field of thoracic surgery there are open and also minimally invasive surgical procedures, during which actions are performed on the patient's thorax while keeping the ribs essentially intact. This implies high difficulty in manoeuvring during the procedure, as well as a high risk of complications and a longer and more complicated post-operative period for the patient.

In particular, the procedure related to the correction of a pathology such as *pectus excavatum* in a patient is currently performed by means of a surgical procedure, which is generally minimally invasive.

During this procedure, an element is inserted to correct the deformity associated with the pathology, which must remain in position for a long period of time after the operation.

Thus, the insertion of said element is, at present, a complicated task given that there is little room for manoeuvre during the procedure and the space available is limited by rigid elements, such as the patient's own rib cage.

In addition, the positioning of the element inserted during the procedure is demanding. The maintenance of said proper position for the element inside the rib cage is necessary. However, once the element is inserted, it is subjected to high stresses, which can cause said element to be moved and/or displaced, leading to failure of the intervention. In these cases, additional surgery is required to reposition and/or remove the inserted element, which means that treatment is prolonged, painful and difficult for the patient.

To avoid this problem, the present technical field sometimes opts for open surgeries, which allow more room for manoeuvre and space for the insertion of external elements into the patient's rib cage.

However, this involves a more physically demanding procedure, with longer recovery time and additional incisions that cause not only more pain for the patient, but also a higher risk of injury to the intercostal vessels or nerves around the ribs, as well as a considerably worse cosmetic result due to scarring. Solutions such as the one shown in CN 113440211 A are an example of said physical demand, since the manoeuvre of the device requires a direct push on the bone elements, resulting in a complete cut and not a break, which increases the adverse effects on the patient.

Additionally, in cases where one or more of the patient's ribs are cut during the surgical intervention, this cutting is performed using highly invasive devices in the patient's rib cage, and individually for each rib with sharp-edged elements, resulting in long and painful patient recoveries.

It is also the case that currently available devices for bone cutting, particularly of ribs, are highly unsafe and physically demanding instruments. In addition, the bone cut in each case is different, which leads to inefficient cutting in many cases.

### DESCRIPTION OF THE INVENTION

The present invention solves the aforementioned problems by means of a device aimed at bone breaking, in particular breaking the ribs of a patient's rib cage.

That is, the present device is designed to break a plurality of bone elements, that is, a plurality of ribs of the patient's rib cage in a single manoeuvre, particularly a single simple percutaneous manoeuvre.

The manoeuvre performed allows the consecutive cutting of a plurality of adjacent ribs during the surgical procedure of inserting external elements into the patient's rib cage.

In this way, a first inventive aspect is a *device for breaking ribs, comprising:*
- *a main body, which in turn comprises:*
   ∘ a *structure comprising a first end, a second end and a hollow channel extending in a longitudinal direction X-X' from the first end to the second end, and*
   ∘ *support means,*
- *a handle comprising a rod extending along the longitudinal direction X-X', wherein the rod comprises a first section and* a *second section,*
- *guiding means comprising a through hole, wherein the through hole at least partially houses the first section of the rod of the handle and is configured so that said first section of the rod of the handle rotates therein,*
- *cutting means extending at least partially along the longitudinal direction X-X*',
*wherein the cutting means are coupled to the second section of the rod of the handle, and wherein rotation of the first section of the rod of the handle causes the cutting means to move over the second section of the rod of the handle along the longitudinal direction X-X'.*

In this way, the device is configured by means of a main body, which allows the rest of the elements to be assembled thereon, as said main body is rigid, preferably metal or plastic.

In turn, the main body comprises a structure and support means, coupled to said structure. Preferably, the structure and support means are integral, manufactured in a single piece.

The structure has a first end and a second end, defining the dimensions of the main body, in particular its overall length. This length is defined along a longitudinal direction X-X', along which both the structure and the main body extend.

In a particular embodiment, the support means are two protrusions, symmetrically located on both sides of the longitudinal direction X-X'. In a particular embodiment, at least one of the protrusions extends along a transverse direction Y-Y', perpendicular to the longitudinal direction X-X'. In a particular embodiment, these protrusions are cylinders.

Additionally, the structure of the main body also comprises a hollow channel, which also extends along the longitudinal direction X-X'. In a particular embodiment, the hollow channel is located between the two protrusions that configure the support means.

In a particular embodiment, the hollow channel delimits a space on the structure, which allows several elements to be housed therein. Advantageously, this allows the required elements of the device to be housed and protected.

In a particular embodiment, the hollow channel has a square cross-section.

Furthermore, the device according to the first inventive aspect also comprises a handle, which in turn comprises a rod extending along the longitudinal direction X-X', parallel to the structure of the main body.

Said rod comprises a first section and a second section, both cylindrical, wherein the first section is a section configured to rotate around the longitudinal direction X-X', as is the second section, which is also configured to rotate rigidly connected to the first section. In a particular embodiment, the second section of the rod is threaded.

Said first and second sections are located one after the other in the longitudinal direction X-X', being coupled and/or connected together. In a particular embodiment, the first section and the second section are integral, being made in one piece.

In a particular embodiment, the handle further comprises a grip, preferably hollow, which is connected to the rod, in particular to the first section of the rod. That is, the grip and the first section of the rod are fastened to each other. Advantageously, said grip provides a gripping surface to rotate the rod, in particular to rotate the first section of the rod.

In a particular embodiment, the grip extends along the transverse direction Y-Y', perpendicular to the longitudinal direction X-X'. In a particular embodiment, the grip is essentially hollow.

In a particular embodiment, the rod further comprises a third section, also cylindrical, configured to be joined to the handle. In a particular embodiment, the third section is located after the first section, at the opposite end to the second section; therefore, the first section, the second section and the third section are coaxial to each other.

In a particular embodiment, the diameter of the second and third sections of the rod are the same, while the diameter of the first section of the rod is larger than that of the second and third sections of the rod. Advantageously, this allows the rod to rotate uniformly.

In a particular embodiment, the first section and the second section of the rod of the handle and/or the third section are integrally configured in a single piece. Advantageously, this allows the rotational movement exerted on the first section to be transferred to the second section. In the particular embodiment wherein the rod comprises a third section, said rotational movement is transmitted from the third section to the first section and from this section to the second section.

Additionally, the device also comprises guiding means. Said guiding means comprise a through hole, wherein the through hole at least partially houses the first section of the rod of the handle. In turn, the hole is configured such that the first section of the rod of the handle rotates therein.

That is, the rotational movement exerted on the rod, preferably via the grip and the third section of the rod, is transformed into a rotation of the first section of the rod inside the hole of the guiding means. Advantageously, this allows the guiding means to move along the hollow channel, that is, parallel to the longitudinal direction X-X' of the structure of the main body when the first section of the rod rotates about the longitudinal direction X-X' inside the guiding means.

In a particular embodiment, the guiding means are fastened to the structure of the main body.

In a particular embodiment, the guiding means are configured in two parts connected together, wherein the through hole is partially located in each of the parts. In this way, the first section of the rod is housed in the through hole which is created after the two parts of the guiding means are connected together, so that both parts of the guiding means partially clasp the first section of the rod.

In a particular embodiment, the through hole of the guiding means comprises at least two sections with different dimensions, these being cylindrical sections.

In a particular embodiment, the first section of the rod of the handle comprises at least two sections, both cylindrical, wherein the two sections of the through hole and the two sections of the first section are complementary.

Advantageously, this allows the first section, second section and/or third section of the rod to be housed in each of the different sections of the guiding means, the entire length of the housing of the rod thus being more tightly fitted to the guiding means and allowing said sections to be rotated on the appropriate path.

Additionally, the device comprises cutting means extending at least partially along the longitudinal direction X-X', wherein said cutting means are coupled to the second section of the rod of the handle. Said coupling is detachable.

Preferably, the cutting means comprise a threaded hole that couples to the second threaded section of the rod, the coupling between the two elements thus being a threaded connection. Said threaded hole is preferably a through hole along a portion of the cutting means.

In this way, in the operating situation of the device it is ensured that the rotation of the first section of the rod of the handle causes the cutting means to move on the second section of the rod of the handle along the longitudinal direction X-X'.

That is, in a particular embodiment, the rotation of the grip of the handle transmits the rotational movement to the first and second section of the rod, which rotates inside the guiding means.

In turn, the rotation of the second section of the rod produces a translational movement of the cutting means along the hollow channel of the main body. Thus, the cutting means are configured to move along the length of the hollow channel of the body, while the guiding means remain fixed, acting as a stop along the path of the cutting means and cooperating with the rotational movement of the different sections of the rod.

In a particular embodiment, the cross section of the cutting means is complementary in at least one portion to the cross section of the hollow channel of the main body. Advantageously, this allows a homogeneous sliding along the longitudinal direction X-X' of the hollow channel, which in turn does not deviate as there is no clearance between the guiding means and the inner face of the hollow channel through which said guiding means slide.

In this way, the rotation of the first section of the rod in turn causes the second section of the rod to rotate, which in turn causes the cutting means to move along the longitudinal direction X-X'.

In a particular embodiment, the cutting means comprise a portion extending in a transverse direction Y-Y', perpendicular to the longitudinal direction X-X', wherein said portion comprises a sharp edge, which allows a directed breakage of the bone element, in particular of the rib. Thus, in this particular embodiment, the cutting means are configured in an L-shape, the sharp edge being located in the lower portion of the cutting means (and therefore of the device) according to the longitudinal direction X-X', and said sharp edge extending according to the transverse direction Y-Y'.

Lastly, the device further comprises first resting means and/or second resting means, wherein:
- the first resting means are positioned on the support means of the main body and configured to rotate about said support means,
- the second resting means extend along the Y-Y' direction. Furthermore, the second resting means are coupled to the structure of the main body via connection means.

In a particular embodiment, the first resting means are configured as cylinders, supported on the support means such that said support means are housed inside the first resting means.

In a particular embodiment, the first resting means comprise drive means configured to apply external force on the first resting means.

Advantageously, this allows the first resting means to be moved and/or rotated on the support means.

In a particular embodiment, the drive means are levers located on the outer surface of the cylindrical rollers forming the first resting means. Said levers, by means of the drive thereof, allow the rollers to rotate about the longitudinal axis of the support means. This, in turn, causes the rollers to partially rotate on an external surface on which they bear.

In a particular embodiment, the second resting means are configured as protrusions, which extend along the transverse direction Y-Y', perpendicular to the longitudinal direction X-X', said protrusions thus defining a support surface along the said transverse direction Y-Y'. In an operational situation, the protrusions are configured to be supported on the outer surface of at least one of the bone elements, that is, at least one of the ribs. In this way, the second resting means are L-shaped, wherein the coupling to the structure is performed via a connecting rod, which extends along the longitudinal direction X-X'.

In a particular embodiment, the coupling between the second resting means, via the connecting rod, and the structure of the main body is by means of bolted connections.

In a particular embodiment, the second resting means are configured in the form of two parts that are symmetrical in the longitudinal direction X-X', the support surfaces thereof extending along the transverse direction Y-Y'.

Advantageously, the device according to the first inventive aspect allows in an operational situation, that is, fully assembled and in a surgical procedure situation of a patient, the quick breakage of the consecutive bone elements, incorporating only an additional incision on the patient's thorax for the insertion of the device inside the thoracic cavity of the patient during said procedure.

In this way, breaking the bone elements using the present device not only facilitates the surgical procedure in general but also improves the insertion of external elements into the patient's thoracic cavity through the space created by the breakage, as well as improving the breaking method itself of said bone elements.

### DESCRIPTION OF THE FIGURES

To complete the description, and for the purpose of helping to make the features of the invention more readily understandable, this specification is accompanied by a set of figures constituting an integral part of the same, which by way of illustration and not limitation represent the following:
Figures 1A and 1B show a perspective and a cross-sectional view, respectively, of a particular embodiment of a device according to the first inventive aspect.
Figure 2 shows a particular embodiment of the main body of the device shown in figures 1A and 1B.
Figure 3 shows a particular embodiment of the handle of the device shown in figures 1A and 1B.
Figure 4 shows a particular embodiment of the second support means of the device shown in figures 1A and 1B.

### PREFERRED EMBODIMENT OF THE INVENTION

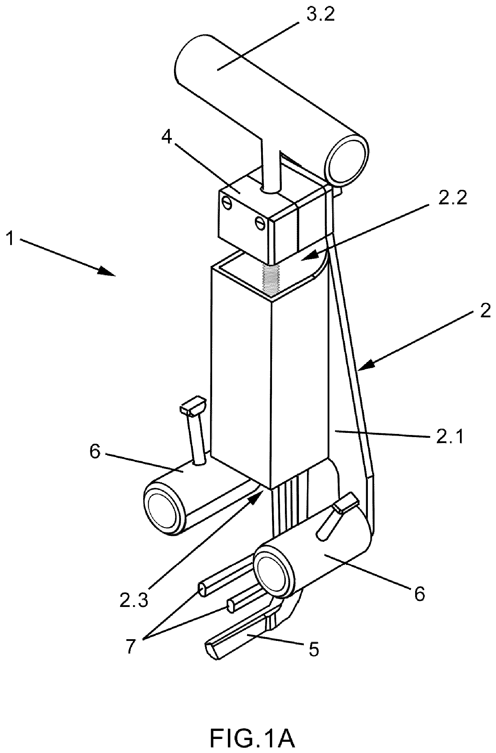

Figure 1A shows a perspective view of a particular embodiment of a device (1) for breaking ribs.

Said device (1) is configured by means of a main body (2), with a flat and symmetrical structure (2.1), which acts as a support wall for the rest of the elements belonging to the device (1). In addition, the structure (2.1) is configured in a prismatic shape, which encloses a hollow channel (2.4) which in turn houses several elements also belonging to the device (1). The structure (2.1) is made of metal, from a die-cut and folded sheet that allows the appropriate shape to be given to each of its parts. The connection between elements of the structure (2.1) itself is performed by welding. In other particular embodiments, the structure (2.1) is obtained directly by additive manufacturing, avoiding connections between elements, or also by a machined billet.

Said structure (2.1) further comprises a first end (2.2) and a second end (2.3), which delimit the start and end of the hollow channel (2.4). Thus, the first end (2.2) is located in the upper portion of the device (1) as shown in Figure 1A, while the second end (2.3) corresponds to the bottom portion of the device (1).

The device (1) of figure 1A additionally shows guiding means (4), coupled to the first end (2.2) of the main body (2), by means of screwed connections. Likewise, figure 1A shows a handle (3) partially housed in the hollow channel (2.4), as well as partially housed in the guiding means (4). The handle (3) can be actuated via the grip (3.2), which protrudes from the guiding means (4) and has a configuration that allows it to be gripped by a user.

As can be seen, the grip (3.2) is cylindrical and hollow, which helps to reduce the weight of the device (1).

Additionally, the main body (2) also comprises, on the side of the second end (2.3) thereof, support means (2.5), configured in the form of two cylinders that project from the main plane of the structure (2.1). There are first resting means (6) housed on said cylinders (2.5), which are also cylindrical, which further comprise, connected to the outer surface thereof, drive means in the form of a lever, which allows the partial rotation of the first resting means (6) on the surface on which they rest.

There are also second support means (7) on the side of the second end (2.3) of the main body (2), which are fixed to the structure (2.1) by means of screwed connections, and which are also configured in the form of protrusions perpendicular to the main plane of said structure (2.1), in the form of prismatic protrusions with a flat face, on which to bear on a surface of the patient, in particular on the outer surface of the ribs to be broken.

Lastly, figure 1A also shows, on the side of the second end (2.3) of the main body (2), cutting means (5) configured in the form of an L-shaped cutter, that is, with a protrusion perpendicular to the main plane of the structure (2.1). Said protrusion comprises a sharp edge with which to break the ribs in the longitudinal direction X-X' of the device (1).

As can be seen, the device (1) is symmetrical according to its main longitudinal plane (X-X', Y-Y').

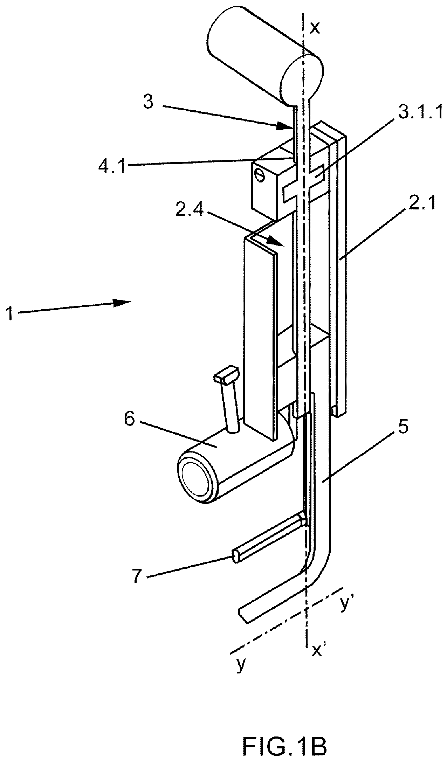

Figure 1B shows a longitudinal section of the device (1) of figure 1A. As can be seen, said device (1) extends, as indicated above, in a longitudinal direction X-X'. Thus, the structure (2.1) is symmetrically configured on both sides of said longitudinal direction X-X'.

It can also be seen how the rod (3.1) of the handle (3) extends along said longitudinal direction X-X', the first section (3.1.1) of said rod (3.1) being housed inside the through hole (4.1) of the guiding means (4).

Likewise, the second section (3.1.2) of the rod (3.1) extends through the inside of the hollow channel (2.4) and is threaded to the cutting means (5), in particular to the side opposite the protrusion of said cutting means (5).

In the operational state, as shown in figure 1B, the cutting means (5) move through the inside of the hollow channel (2.4) in the longitudinal direction X-X', thus having sufficient travel between their two end positions to break the established ribs.

In particular, the guiding means (4) are configured from two prismatic pieces comprising a groove on one of the sides thereof, along the entire length thereof, with two sections with different widths. When said pieces are facing each other on the face containing the groove, a through hole (4.1) is formed, which houses in its first section the first section (3.1.1) of the rod (3.1), and in the second section it houses on one side the second section (3.1.2) of the rod (3.1) and the third section (3.1.3) of the rod (3.1) on the other side, which is connected to the grip (3.2) by means of welding, adhesive or obtained by additive manufacturing avoiding the connection means.

Lastly, it can also be seen how the support means (2.5) and the first support means (6) that are coupled on them, as well as the second support means (7) and the protrusion in the form of a cutter of the cutting means (5) extend in the transverse direction Y-Y', perpendicular to the longitudinal direction X-X'.

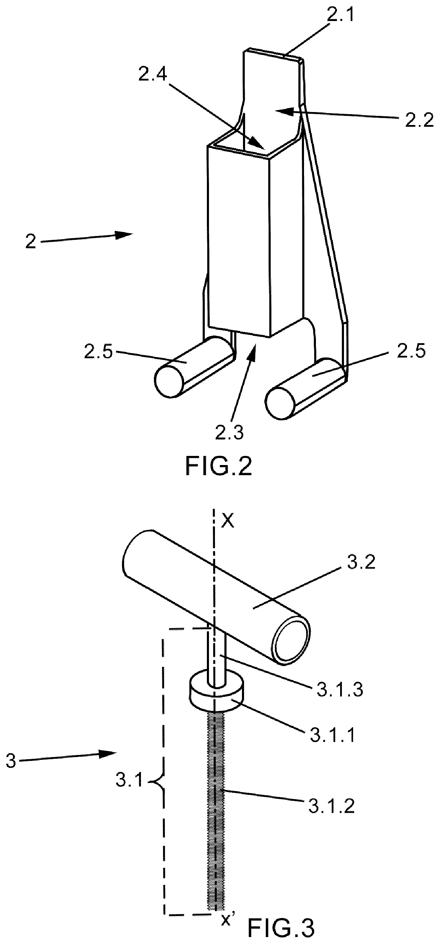

Figures 2, 3 and 4 show, in detail, the configuration of some of the elements referred to above.

For example, Figure 2 shows the main body (2) of the device (1) of figures 1A and 1B, such that the configuration of the structure (2.1), obtained on the basis of any of the aforementioned manufacturing processes, is seen, such that a support wall is obtained from which a prismatic shape, with a square cross section, is projected and which configures the hollow channel (2.4).

Likewise, the structure (2.1) comprises, at its second end (2.3), two wings from which the protrusions that configure the support means (2.5) project, said protrusions being cylindrical and perpendicular to the structure (2.1).

Furthermore, figure 3 shows the handle (3) of the device (1) of figures 1A and 1B.

Said handle (3) is configured by the rod (3.1), which extends along the longitudinal direction X-X', and which is made of a third section (3.1.3) that is cylindrical and smooth, and a first section (3.1.1) adjacent to the third section (3.1.3). The first section (3.1.1) is also cylindrical, but larger in cross-section than the third section (3.1.3). Next, following the same longitudinal direction X-X', is the second section (3.1.2), also cylindrical and threaded, and with the same cross-section as the third section (3.1.3).

At its opposite end, the third section (3.1.3) is coupled to the grip (3.2) of the handle (3).

As shown, the first (3.1.1), second (3.1.2) and third (3.1.3) sections are integrally configured in one piece.

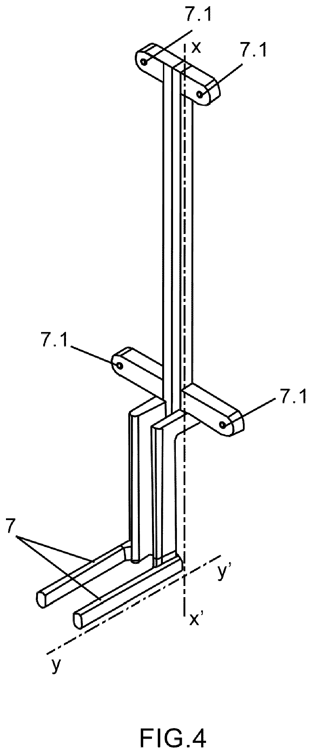

Lastly, figure 4 shows the second support means (7) of the device (1) of figures 1A and 1B.

Thus, the second support means (7) are configured as a symmetrical element with respect to the median plane (X-X', Y-Y'), and comprises two protrusions extending in the transverse direction Y-Y', perpendicular to the longitudinal direction X-X'. These protrusions have a prismatic cross section and have a flat face that allows them to bear on a surface.

Additionally, as shown, the second support means (7) also comprise four lugs, symmetrical with respect to the longitudinal direction X-X', which in turn comprise screwed connections (7.1) to the structure (2.1) of the main body.

The second support means (7) are fastened to the structure (2.1) by means of said screwed connections (7.1).

In an operational situation, the device (1) shown in figure 1A is manipulated so as to break a plurality of adjacent ribs consecutively. In this way, the device is inserted into the chest cavity of a patient, either supported by the first support means (6) on the skin of the patient's thorax, or supported by the second support means (7) below the skin, but on the outer surface of the ribs to be broken.

In either case, the rotation of the handle (3) by turning the grip (3.2) allows the cutting means (5) to move from the second end (2.3) to the first end (2.2) of the main body (2), breaking the ribs by means of the sharp edge of the cutter of the cutting means (5) in its path.

This single manoeuvre is performed in such a way that the sharp edge of the cutting means (5) is positioned perpendicular to the ribs to be broken.

Additionally, the first (6) and/or second (7) support means help to keep the device (1) in position, as well as to exert the necessary pressure of the device without exerting a direct force on the ribs of the patient other than the shearing force or bending force exerted by the sharp edge of the cutting means (5).

Lifting of the cutting means (5) through the inside of the hollow channel (2.4) is achieved by rotating the grip (3.2) of the handle (3), which is transmitted to the first section (3.1.1) of the rod (3.1) inside the through hole (4.1) of the guiding means (4). In turn, this rotational movement is also transmitted to the second section (3.1.2) of the rod (3.1) which, via the thread machined thereon, transforms this rotational movement into a translational movement of the cutting means (5) along said second section (3.1.2) of the rod (3.1), according to the longitudinal direction X-X'.

## Claims

1. A device (1) for breaking ribs, comprising:
- a main body (2), which in turn comprises:
∘ a structure (2.1) comprising a first end (2.2), a second end (2.3) and a hollow channel (2.4) extending in a longitudinal direction X-X' from the first end (2.2) to the second end (2.3), and
∘ support means (2.5),
- a handle (3) comprising a rod (3.1) extending along the longitudinal direction X-X', wherein the rod (3.1) comprises a first section (3.1.1) and a second section (3.1.2),
- guiding means (4) comprising a through hole (4.1), wherein the through hole (4.1) at least partially houses the first section (3.1.1) of the rod (3.1) of the handle (3) and is configured so that said first section (3.1.1) of the rod (3.1) of the handle (3) rotates therein,
- cutting means (5) extending at least partially along the longitudinal direction X-X',
wherein the cutting means (5) are coupled to the second section (3.1.2) of the rod (3.2) of the handle (3), and wherein the rotation of the first section (3.1.1) of the rod (3.1) of the handle (3) causes the cutting means (5) to move over the second section (3.1.2) of the rod (3.2) of the handle (3) along the longitudinal direction X-X'.

2. The device (1) according to the preceding claim, further comprises first resting means (6) and/or second resting means (7), wherein:
- the first resting means (6) are positioned on the support means (2.5) of the main body (2) and configured to rotate about said support means (2.5),
- the second resting means (7) extend along the longitudinal direction X-X' and are coupled to the structure (2.1) of the main body (2) by means of connecting means.

3. The device (1) according to claim 2, wherein the first resting means (6) comprise drive means configured to apply external force on the first resting means (6).

4. The device (1) according to any of claims 2 or 3, wherein the coupling between the second resting means (7) and the structure (2.1) of the main body (2) is by means of screwed connections (7.1).

5. The device (1) according to any of the preceding claims, wherein the channel (2.4) of the main body (2) has a square cross section.

6. The device (1) according to any of the preceding claims, wherein the handle (3) further comprises a grip (3.2), preferably hollow, which is connected to the rod (3.1) through the first section (3.1.1) of the rod (3.1).

7. The device (1) according to claim 6, wherein the rod (3.1) further comprises a third section (3.1.3) configured to be connect to the handle (3.2).

8. The device (1) according to any of the preceding claims, wherein the first section (3.1.1) and the second section (3.1.2) of the rod (3.2) of the handle (3) and/or the third section (3.1.3) are integrally configured in a single piece.

9. The device (1) according to any of the preceding claims, wherein the second section (3.1.2) of the rod (3.2) of the handle (3) is threaded.

10. The device (1) according to any of the preceding claims, wherein the guiding means (4) are configured in two parts connected together, wherein the through hole (4.1) is partially located in each of the parts.

11. The device (1) according to any of the preceding claims, wherein the through hole (4.1) of the guiding means comprises at least two cylindrical sections.

12. The device (1) according to claim 11, wherein the first section (3.1.1) of the rod (3.1) of the handle (3) comprises two cylindrical sections, wherein the two sections of the through hole (4.1) and the two sections of the first section (3.1.1) are complementary.

13. The device (1) according to any of the preceding claims, wherein the coupling of the cutting means (5) to the second section (3.1.2) of the rod (3.2) of the handle (3) is by means of a threaded connection.

14. The device (1) according to any of the preceding claims, wherein the cutting means (5) comprise a portion extending in a transverse direction Y-Y', perpendicular to the longitudinal direction X-X', wherein said portion comprises a sharp edge.

15. The device (1) according to any of the preceding claims, wherein the section of the cutting means (5) in at least one portion of said cutting means (5) is complementary to the section of the channel (2.4) of the main body (2).
